# EUROPÄISCHE PATENTSCHRIFT

(11) **EP 1 023 059 B1**
(45) Veröffentlichungstag und Bekanntmachung des Hinweises auf die Patenterteilung: **21.12.2005**
(21) Anmeldenummer: 98961013.4
(22) Anmeldetag: 16.10.1998
(51) Int. Cl.: A61K 31/21

(54) **PHARMAZEUTISCHE ZUBEREITUNGEN**
PHARMACEUTICAL PREPARATIONS
PREPARATIONS PHARMACEUTIQUES

(30) Priorität: 16.10.1997 DE 19745618
(43) Veröffentlichungstag der Anmeldung: 02.08.2000
(73) Patentinhaber: Alpharma-Isis GmbH & Co. KG, 40764 Langenfeld (DE)
(72) Erfinder: GÜNTHER, Ulrich, D-08146 Mülsen St. Niclas (DE); FREITAG, Sabine, D-08056 Zwickau (DE); KUNTZE, Ulrike, D-08468 Heinsdorfergrund (DE); LEHR, Wilhelm, Thomas, D-08432 Steinpleis (DE)
(74) Vertreter: Adrian, Albert
(86) Internationale Anmeldenummer: PCT/DE1998/003030
(87) Internationale Veröffentlichungsnummer: WO 1999/018930

(56) Entgegenhaltungen:
- EP-A2- 0 104 877
- EP-B1- 0 413 694
- WO-A1-94/01103
- DE-A1- 3 328 094
- DE-A1- 4 410 997
- US-A- 3 028 307
- US-A- 4 369 172
- Database WPIL ON Questel, Woche 8709, London: Derwent Publications Ltd., AN 87-062702, Klasse A61K; XP002900400 & RO 90251 A (TERAPIA INTR MEDICAMENTE), Zusammen- fassung.

## Beschreibung

### Anwendungsgebiet der Erfindung

Die vorliegende Erfindung betrifft neue, insbesondere peroral applizierbare, pharmazeutische Zubereitungen, die als Arzneistoff Pentaerithrityltetranitrat (PETN) oder seine Metaboliten in solchen Mengen enthalten, die nach Einmalgabe (pro Tag) die ausreichende Versorgung des Patienten garantieren.

### Bekannter technischer Hintergrund

Organische Salpetersäureester, auch als organische Nitrate bezeichnet, wie Glyceroltrinitrat (GTN) (Murrel, Lancet: 80, 113, 151 (1879)), Pentaerythrityltetranitrat (PETN) (Risemann et al., Circulation, Vol. XVII, 22 (1958), US-PS-2370437), Isosorbid-5-mononitrat (ISMN) (DE-OS-2221080, DE-OS-2751934, DE-OS-3028873, DE-PS-2903927, DE-OS-3102947, DE-OS-3124410, EP-A1-045076, EP-A1-057847, EP-A1-059664, EP-A1-064194, EP-A1-067964, EP-A1-143507, US-PS-3886186, US-PS-4065488, US-PS-4417065, US-PS-4431829), Isosorbiddinitrat (ISDN) (L. Goldberg, Acta Physiolog. Scand. 15, 173 (1948)), Propatylnitrat (Médard, Mem. Poudres 35: 113 (1953)), Trolnitrat (FR-PS 984523) oder Nicorandil (US-PS-4200640) und ähnliche Verbindungen sind Vasodilatatoren, die zum Teil seit Jahrzehnten schwerpunktmäßig bei der Indikation Angina pectoris bzw. koronarer Herzkrankheit (KHK) breitesten therapeutischen Einsatz finden (Nitrangin®, Pentalong®, Monolong®, u.a.). Die DE 4 410 997 betrifft die Verwendung von stickstoffmonoxidfreisetzenden und/oder - übertragenden Verbindungen, von Stimulatoren der endogenen Stickstoffmonoxidbildung sowie von Stimulatoren der Guanylatzyklase zur Prävention, Behandlung und Beseitigung endothelialer Dysfunktionen sowie von Erkrankungen, welche durch endotheliale Dysfunktionen hervorgerufen werden oder mit diesen einhergehen.
Schließlich wird in diesem Zusammenhang die EP-A1- 0413694 genannt, deren Gegenstand pharmazeutische herzschützende Zusammensetzungen sind, die Amiooadaron, ein Nitroderivat, nämlich Isosorbiddinitrat und gegebenenfalls einen Betablocker enthalten.

Ausgehend von den pharmakokinetischen Eigenschaften sowie der Applikationsart (oral, sublingual, transdermal) unterscheidet man zwischen Akut- und Langzeitnitraten. Akutnitrate wie z. B. Glyceroltrinitrat werden zur Anfallsbehandlung der Angina pectoris bzw. vor körperlichen Belastungen oder anderen Situationen, die erfahrungsgemäß Angina pectoris-Anfälle auslösen können, eingesetzt. Darüber hinaus sind diese beim akuten Myokardinfarkt, bei akuter Linksherzinsuffizienz und katheterinduzierten Koronarspasmen bei Koronarangiographie indiziert. Langzeitnitrate sind zur Prophylaxe und Langzeitbehandlung der Angina pectoris zugelassen; bei akutem Angina pectoris-Anfall und akutem Myokardinfarkt aufgrund des langsamen Wirkungseintritts hingegen kontraindiziert.

Organische Nitrate besitzen einen hohen Stellenwert in der medikamentösen Behandlung und Prävention verschiedener Krankheiten, die sich nicht allein auf Herz-/Kreislauferkrankungen beschränken. Den meisten der bisher bekannten organischen Nitrate haftet allerdings eine Reihe gewichtiger therapeutischer Nachteile an. So ist z.B. die sogenannte Nitrattoleranz zu beobachten, d.h. die Abnahme der Nitratwirkung bei hoher Dosierung oder bei Applikation längerwirkender Nitrate. Ebenso sind Nebenwirkungen wie Kopfschmerzen, Schwindel, Übelkeit, Schwächegefühl, Hautrötung sowie die Gefahr eines stärkeren Blutdruckabfalls mit reflektorischer Tachykardie belegt (Mutschler, "Arzneimittelwirkungen", Wissenschaftliche Verlagsgesellschaft mbH, Stuttgart (1991)). Demgegenüber besitzt PETN als Arzneistoff eine Reihe herausragender Eigenschaften, insbesondere geringes bis fehlendes Auftreten der oben genannten Nebenwirkungen, welche eine bevorzugte Verwendung dieser Verbindung als Pharmakon gegenüber anderen organischen Nitraten begründen (Schriftenreihe "Pentaerythrityltetranitrat", Dr. Dietrich Steinkopff Verlag, Darmstadt (1994 bis 1997)).

Der Arzneistoff PETN gehört zu den ältesten und am längsten für die Therapie kardiovaskulärer Erkrankungen eingesetzten Langzeitnitraten. PETN ist ein typischer Vertreter aus der Stoffgruppe der sogenannten organischen Nitrate. Organische Nitrate sind Prodrugs, aus denen nur in bestimmten Zelltypen des Organismus (bes. Endothel- und glatte Gefäßmuskelzellen) über enzymatische Bioaktivierungsvorgänge radikalisches Stickstoffmonoxid, NO, als eigentliche Wirksubstanz freigesetzt wird. Im Gefäßbereich vermittelt NO hauptsächlich die Relaxation von Arterien und Venen und ist Hauptregulator der Vasomotion beispielsweise in den Koronararterien. Organische Langzeitnitrate wie PETN entfalten daher entsprechende vasodilatatorische Wirkungen, die therapeutisch, z.B. zum Zweck der Behandlung der ischämischen Durchblutungsstörungen des Herzens, sinnvoll ausgenutzt werden. Ihr Einsatz erscheint aus pharmakologischer Sicht dann besonders sinnvoll, wenn krankheitsbedingt ein relativer Mangel an NO besteht. Molekularpharmakologisch bedeutsam für PETN ist außerdem die spezifische Aktivierung des Enzyms der löslichen Guanylatzyklase, das in glatten Gefäßmuskelzellen und Thrombozyten angetroffen wird. Dieses Enzym katalysiert die Bildung von zyklischem Guanosinmonophosphat (cGMP), das als eigentlicher Mediator der Vasorelaxation anzusehen ist. Zudem wird hierdurch auch eine pathologisch erhöhte Thromboseneigung günstig beeinflußt. PETN ist als Langzeitnitrat mit eigenständigem pharmakologischen Profil charakterisiert. Diese Eigenständigkeit bezieht sich in erster Linie auf die gute Verträglichkeit bei vergleichbar starker hämodynamischer Wirksamkeit, die ausgeprägte Venoselektivität, die gefäßprotektiven bzw. antiatherosklerotischen Eigenschaften und das pharmakokinetische Verhalten, das durch einen enterohepatischen Kreislauf gekennzeichnet ist. Die Analyse und Bewertung der pharmakokinetischen Daten zeigt, dass der Metabolit Pentaerythrityldinitrat im wesentlichen für die Akutwirkung und der Metabolit Pentaerythritylmononitrat für die Langzeitwirkung von PETN verantwortlich ist.

Die galenische Verarbeitung der organischen Nitrate zu pharmazeutischen Zubereitungen zur Behandlung von Angina pectoris bzw. der ischämischen Herzkrankheit sind allgemein bekannt. Für Arzneimittel mit diesen Indikationen ist vor allem die perorale, parenterale, sublinguale oder transdermale Applikation in Form von Tabletten, Dragees, Kapseln, Lösungen, Sprays oder Pflastern beschrieben (DD-A5-293492, DE-AS-2623800, DE-OS-3325652, DE-OS-3328094, DE-PS-4007705, DE-OS-4038203, JP-Anmeldung 59/10513 (1982)). Darüber hinaus ist eine Vielzahl von Arzneimitteln auf der Basis organischer Nitrate in ausschließlich retardierter Form mit länger anhaltender Wirkung bekannt. Diese Depotarzneiformen weisen besonders bei medikamentöser Dauertherapie wesentliche Vorteile auf, wie Erreichen eines relativen konstanten Arzneistoffspiegelbildes im Blut und damit Sicherung einer gleichmäßigen Wirksamkeit und der Verringerung von Nebenwirkungen. Die Anwendung höherer Nitratdosierungen für die Verabreichung einer gesamten Tagesdosis macht eine Retardierung der Arzneistoffe aus therapeutischer Sicht zwingend erforderlich. Einerseits muß aufgrund der auftretenden, das Befinden des Patienten stark beeinträchtigenden Nebenwirkungen eine höhere Nitratkonzentration durch sichere Liberationssteuerung unbedingt vermieden werden. Andererseits verbietet sich eine unretardierte Gabe hoher Nitratdosen, um die Wirkungsabnahme infolge der Nitrattoleranz zu vermeiden (Mutschler, "Arzneimittelwirkungen", Wissenschaftliche Verlagsgesellschaft mbH, Stuttgart (1991), S. 418 ff.). Im Falle von peroral applizierbaren Arzneiformen können Retardarzneimittel die notwendige Einnahmefrequenz beträchtlich reduzieren, im günstigen Fall bis auf 1 Tagesdosis, und verbessern damit wesentlich die Patienten-Compliance und entlasten sowohl den Patienten als auch den Arzt und das Pflegepersonal in ambulanten und stationaren Einrichtungen. Die Formulierung von Retardarzneimitteln mit gesteuerter Arzneistoffliberation ist hinreichend bekannt. Die Verlängerung der Wirkdauer kann prinzipiell auf chemischem Weg (Veränderung am Arzneistoff), durch pharmazeutisch-technologische Maßnahmen (Formulierung geeigneter Arzneiformen) oder durch Nutzung physiologischer bzw. pharmakologischer Möglichkeiten am Individuum erfolgen. Der Protrahierung der Arzneimittelwirkung durch galenische Maßnahmen kommt bei weitem die größte Bedeutung zu aufgrund vielfältiger Realisierungsmöglichkeiten; diese breite Variation bezieht sich sowohl auf Arzneiformen (z.B. Tabletten, Filmtabletten Pellets u.a.) als auch auf Retardierungsverfahren (Überzug, Einbettung, Matrixbildung u.a) und nicht zuletzt auf notwendige, die Liberation steuernde Retardierungshilfsstoffe (z.B. Cellulosederivate, Acrylsäurecopolymere, verschiedene Plastmaterialien, Fette, Wachse, Ionenaustauscher). Bisher wird die Einmalgabe von organischen Nitraten ausschließlich durch eine Verlängerung der Arzneistoffliberation erreicht, d.h. eine einmal tägliche Gabe bei gleichzeitiger Vermeidung sowohl subtherapeutischer als auch toxischer Plasma- bzw. Gewebskonzentrationen sowie Aufrechterhaltung optimaler therapeutischer Gewebskonzentrationen war bisher bei organischen Nitraten (z. B. Isosorbidmono- und Isosorbiddinitrat) nur durch Verzögerung der Arzneistofffreisetzung auf galenischem Wege zu erreichen. Derartige retardierte Zubereitungen lassen sich im allgemeinen nur aufwendig herstellen. Oftmals müssen mehrstufige technologische Verfahren angewendet werden, um die konkreten galenischen Formen mit dem gewünschten Liberationsprofil über einen längeren Zeitraum zu garantieren. Daneben sind auch prinzipielle Nachteile bei der peroralen Applikation von retardierten Zubereitungen wie unverdaute Ausscheidung, unvollständige Arzneistoffliberation, Komplikationen bei Nichteinhaltung der Einnahmevorschrift, lokale Irritationen, Beeinträchtigungen der Wirksamkeit durch fettreiche Nahrung bzw. andere Krankheiten des Patienten bekannt (Sucker et al., "Pharmazeutische Technologie", 2. Aufl. (1991); 369 ff.). Bei einigen Arzneistoffen kann man auf diese Maßnahmen verzichten, wenn bereits die chemischen Eigenschaften der Substanz (Salzbildung, Prodrugbildung) eine Verlängerung der Wirkungsdauer ermöglichen. Bisher hat man jedoch bei organischen Nitraten auf diese pharmazeutisch-technologischen Maßnahmen, wie bereits ausgeführt, nicht verzichten können.

Weiterhin ist neben den langjährig bekannten Anwendungen nitrosierend wirkender Substanzen deren Verwendung zur Behandlung und Prävention von Erkrankungen beschrieben, welche ihre Ursache in pathologisch erhöhten Konzentrationen schwefelhaltiger Aminosäuren in Körperflüssigkeiten haben. Diese Krankheitszustände, hervorgerufen durch angeborene oder erworbene Defekte im Metabolismus dieser Aminosäuren und die durch erhöhte Blut- und Urinkonzentrationen besagter Aminosäuren (Homocystinurie) charakterisiert sind, werden unter dem Begriff Homocysteinämie zusammengefaßt (WO-A1-92/18002).

Weitere Verwendungen der vorstehenden Substanzen wurden kürzlich beschrieben, so als endothelprotektive Mittel (DE-A1-4410997), als Mittel gegen pathologisch erhöhten Augeninnendruck (WO-A1-95/13812), als Mittel gegen Dysmenorrhoe, dysfunktionelle uterine Blutungen, vorzeitige Wehentätigkeit bzw. Nachwehen durch Verminderung der uterinen Kontraktilität (WO-A1-95/13802), als Mittel gegen Klimakteriumsbeschwerden (WO-A1-95/13800) oder als Mittel gegen erektile Dysfunktion (Merfort, Münch. Med. Wochenschr. 138 (1996), 504-507; Gomaa et al., Br. Med. J. 312 (1996), 1512-1515).

### Darlegung der Erfindung

Aufgabe der Erfindung ist es, neue, insbesondere peroral applizierbare pharmazeutische Zubereitungen, die als Arzneistoff Pentaerythrityltetranitrat, seine Metaboliten Pentaerythrityltri-, Pentaerythrityldi- oder Pentaerythritylmononitrat enthalten, bereitzustellen, welche bei Einmalgabe einer Dosiseinheit pro Tag den Patienten ohne Einschränkung der Verträglichkeit, ohne Entwicklung von Nitrattoleranzen oder auftretender anderer Nebenwirkungen, ausreichend medizinisch versorgen.

Die Aufgabe der Erfindung wird gelöst durch pharmazeutische Zubereitungen, enthaltend mehr als 80 bis 600 mg Pentaerythrityltetra-, Pentaerythrityltri-, Pentaerythrityldi- oder Pentaerythritylmononitrat und übliche galenische Hilfsstoffe. Hierbei kann die Arzneistoffmenge in weiten Bereichen ohne Verlust der therapeutischen Wirksamkeit und ohne dem Auftreten besonderer Nebenwirkungen variiert werden. Bevorzugt sind die Bereiche mehr als 80 bis 500 mg, 85 bis 500 mg, 100 bis 400 mg, 100 bis 200 mg, 120 bis 180 mg, insbesondere 150 mg. Hierbei kann der Anteil des Arzneistoffs in den Zubereitungen bis zu 50 Masse-%, bevorzugt 10 bis 50 Masse-% betragen. Gemäß einem weiteren Merkmal der Erfindung wird die Aufgabe dadurch gelöst, dass die verwendeten galenischen Hilfsstoffe keine retardierende Wirkung besitzen oder eine solche entfalten, da sie in Mengen eingesetzt werden, die nicht retardierend wirken. Als Hilfststoffe im Sinne dieser Erfindung werden dabei Substanzen verstanden, die es ermöglichen, die Arzneistoffe in die gewünschten Zubereitungsformen zu bringen und die darüber hinaus geeignet sind, die zu erzielenden Eigenschaften der Arzneiform zu erreichen und bekannte zu verbessern, vor allem hinsichtlich der Wirkung der Arzneistoffe, der Wirkungsregulierung, der äußeren Beschaffenheit, der Dosierbarkeit und der Haltbarkeit. Substanzen die als Hilfsstoffe Verwendung finden, müssen folgenden Forderungen genügen: Atoxizitat, therapeutische Indifferenz, physiologische Verträglichkeit, ausreichende Stabilität und Haltbarkeit, Verträglichkeit mit dem Arzneistoff u. ä. Hinsichtlich ihres Verwendungszwecks werden die Hilfsstoffe erfindungsgemäß vorzugsweise aus den Gruppen der Füll-, Binde-, Gleit-, Formentrenn-, Schmier-, Zerfalls-, Feuchthalte-, Adsorbtions-, Retardierungs-, Spreng- oder Gegensprengmittel, Filmbildner, Resorptionsbeschleuniger, Konservierungsstoffe, Stabilisatoren, Emulgatoren, Lösungsvermittler, Salze zur Beeinflussung des osmotischen Drucks, Pufferlösungen, Farb-, Duft-, Aroma- oder Süßstoffe ausgewählt. Verschiedene Hilfsstoffe erfüllen dabei mitunter mehrere Funktionen. Entsprechend der konkreten Aufgabenstellung und der jeweiligen Stoffparameter ist durch den pharmazeutischen Fachmann die geeignete Auswahl von Hilfsstoffen auch hinsichtlich der Vermeidung von Arzneistoff-Hilfsstoff-Inkompatibilitäten zu treffen. Geeignete pharmazeutische Hilfsstoffe sind ohne darauf beschränkt zu sein, Cellulosen, Cellulosederivate, Stärken und modifizierte Stärken Laktose, Sorbit, Gelatine, PVP, Phosphate, Stearinsäure und ihre Salze, Talkum und Siliziumdioxid. Die jeweilige Arzneizubereitung wird bevorzugt in flüssiger oder fester Form bereitgestellt. Hierfür geeignet sind Lösungen, insbesondere zur Zubereitung von Tropfen, Injektionen, Infusionen, Aerosolsprays oder Pulverinhaler, des weiteren Suspensionen, Emulsionen, Sirupe, Tabletten, Filmtabletten, Dragees, Kapseln, Pellets, Pulver, Pastillen, Implantate, Suppositorien, Cremes, Gele, Salben, Pflaster oder andere transdermale Systeme. Überraschenderweise wurde nun gefunden, dass die zur Erreichung des Zieles im Sinne dieser Erfindung verwendeten Hilfsstoffe ausschließlich als Füll-, Spreng- Binde-, Fließregulierungs- bzw. Gleit- und Schmiermittel sowie nicht als liberationssteuemde Mittel fungieren. Dabei sind alle Hilfsstoffe selbst indifferent gegenüber der arzneilich wirksamen Substanz. Erfindungsgemäß werden die genannten pharmazeutischen Zubereitungen insbesondere in fester, peroral applizierbarer Form hergestellt. Hierfür geeignet sind besonders Pulver, Granulate, Pellets, Tabletten, Filmtabletten, Kapseln und Dragees. Die Bereitstellung von galenischen Zubereitungen erfolgt dabei nach den dem pharmazeutischen Fachmann allgemein gelaufigen Arbeitsweisen und - regeln, wobei sich die Auswahl der anzuwendenden Technologien und eingesetzten galenischen Hilfsstoffe in erster Linie nach dem zu verarbeitenden Arzneistoff und dem gewünschten Resorptionsort richtet. Hierbei sind Fragen seiner chemisch-physikalischen Eigenschaften, der gewählten Applikationsform, der gewünschten Wirkungsdauer, des Wirkungsortes sowie der Vermeidung von Arzneistoff-Hilfsstoff-Inkompatibilitäten von besonderer Bedeutung. Es obliegt daher dem Fachmann, anhand bekannter Stoff- und Verfahrensparameter in an sich trivialer Weise Arzneiform, Hilfsstoffe und Herstellungstechnologie auszuwählen. Die erfindungsgemäßen Zubereitungen lassen sich auf Basis einfach anwendbarer Technologien in an sich bekannter Weise vorteilhaft herstellen. Bei der Herstellung der pharmazeutischen Zubereitungen werden Pentaerythrityltetra-, Pentaerythrityltri-, Pentaerythrityldi- oder Pentaerythritytmononitrat in einer geeigneten Hilfstoffmischung in definierten Mengen, so dass die fertiggestellten pharmazeutischen Zubereitung mehr als 80 bis 600 mg des Arzneistoffs enthalten, in einem Mischsystem homogen verteilt. Die entstehenden Pulvermischungen lassen sich bei ausreichend hohem Preßdruck z. B. auf einer Rundlauftablettenpresse zu Tabletten verpressen. Im allgemeinen werden Preßdrucke bis 50 kN ausreichend sein. Bevorzugt sind 10 bis 30 kN. Desweiteren sind die Pulvermischungen auf einer Kapselfüll- und -verschließmaschine in Hartgelatinesteckkapseln oder auf einer Beutelfüllanlage in Sachets abfüllbar. Daneben hat es sich als nützlich erwiesen, die genannten Pulvermischungen in an sich bekannter Weise zunächst zu kompaktieren, zu mahlen und zu homogenisieren, bevor sie anschließend weiter zu Tabletten, Hartgelatinekapseln bzw. Sachets verarbeitet werden. Gemäß einem weiteren Merkmal der Erfindung sind nach dem Eintragen und der homogenen Verteilung des Arzneistoffs in Hilfsstoffmischungen die erhaltenen Pulver auch vorteilhaft mit Bindemittellösungen zu granulieren. Die Granulate können nachfolgend in Kapseln oder Sachets abgefüllt oder zu Tabletten verpreßt werden. Darüber hinaus lassen sich die hergestellten Preßlinge in einer geeigneten Coatinganlage mit einer Hülle überziehen, wobei die so erhaltenen Filmtabletten oder Dragees in der äußeren Schicht ebenfalls nur Hilfsstoffen enthalten, die keine retardierende Wirkung besitzen oder entfalten, wobei als retardierend im Sinne vorliegender Erfindung nicht magensaftresistente Überzüge betrachtet werden. Dabei ist u. a. die homogene Verteilung der erforderlichen Mengen des Arzneistoffes in einer Hilfsstoffmischung ausreichend für das Erlangen des gewünschten therapeutischen Zieles und für die Realisierung der Aufgabenstellung. Besonders die einfache Abfüllung in Kapseln, Beutel, Sachets usw. bzw. die Weiterverarbeitung zu Granulaten oder Tabletten sichert die Anwendungsbreite und Formenvielfalt der erfindungsgemäßen Zubereitungen mit guter Bioverfügbarkeit. Eine weitere Ausgestaltung der Erfindung besteht darin, dass die jeweilige Arzneiform die Gesamtmenge des Arzneistoffs in einer Dosiseinheit inhomogen verteilt, insbesondere in diskreten Teilmengen, enthält. Die erfindungsgemäßen pharmazeutischen Zubereitungen können weiterhin den Einzelarzneistoff, in Kombination mit bekannten Herz-/Kreislauftherapeutika, beispielsweise ACE-Hemmem, Antiatherosklerotika, Antihypertensiva, Betablockern, Cholesterinsenkern, Diuretika, Kalziumantagonisten, Koronardilatatoren, Lipidsenkern, periphere Vasodilatatoren, Thrombozytenaggregations-hemmern oder anderen, ebenfalls als Herz-/Kreislauftherapeutika eingesetzten Substanzen, kombiniert enthalten und ihrer klinischen Verwendung zugeführt werden. Auch die Verabreichung der Kombination in räumlich getrennten Arzneiformen ist möglich und liegt gleichfalls im Umfang der Erfindung. Mit der dargelegten Erfindung werden somit verbesserte und erheblich erweiterte therapeutische Möglichkeiten eröffnet, pathologischen Situationen wie Herz- und Gefäßerkrankungen, insbesondere die koronare Herzkrankheit, Gefäßstenosen und Durchblutungsstörungen der peripheren Arterien, Hypertonie, Mikro- und Makroangiopathien im Rahmen des Diabetes mellitus, Stresszustände in Gefäßen und Geweben wie Atherosklerose sowie die daraus resultierenden Folgekrankheiten, weiterhin erektile Dysfunktion, erhöhten Augeninnendruck, Dysmenorrhoe, dysfunktionelle uterine Blutungen, Dysfunktionen der uterinen Kontraktilität wie vorzeitig einsetzende Wehentätigkeit, Klimakteriumsbeschwerden oder Inkontinenz unter Verwendung der beschriebenen pharmazeutischen Zubereitungen zu behandeln. Die nachfolgenden Beispiele sollen die Erfindung hinsichtlich ihres Wesens und ihrer Ausführung näher erläutern, ohne sie jedoch in ihrem Umfang zu beschränken.

### Ausführungsbeispiele

### Beispiel 1

In einem geeigneten Mischer werden 160 g Pentaerythrityltetranitrat (PETN), 300 g Laktose, 80 g mikrokristalline Cellulose, 76 g Maisstärke, 20 g Talkum, 20 g Siliziumdioxid und 4 g Magnesiumstearat homogen gemischt. Das Mischgut wird bei einer Preßkraft von 10 - 30 kN zu Tabletten mit einer Sollmasse von 600 mg verpreßt.

### Beispiel 2

350 g PETN, 1000 g Laktose, 323 g mikrokristalline Cellulose und 273 g Kartoffelstärke werden in einem Wirbelschichtgranulator gemischt, mit 1050 ml einer 4%igen wäßrigen Stärkelösung granuliert, anschließend getrocknet, gesiebt und mit 60 g Talkum, 20 g Magnesiumstearat sowie 32 g Siliziumdioxid homogen gemischt. Auf einer Rundlauftablettenpresse wird das Granulat bei einer Preßkraft von 10 - 30 kN zu Tabletten mit einer Sollmasse von 1050 mg verpreßt.

### Beispiel 3

In einem geeigneten Mischer werden 900 g Laktose, 300 g Maisstärke, 30 g Siliziumdioxid und 300 g PETN bis zur Homogenität gemischt. Die Mischung wird in Sachets (Beutel) mit 1530 mg Füllgewicht abgefüllt.

### Beispiel 4

In einem Wirbelschichtgranulator werden 450 g PETN, 1350 g Laktose, 300 g mikrokristalline Cellulose und 400 g Kartoffelstärke gemischt. 36 g Gelatine und 18 g Sorbitol, gelöst in 350 g Wasser, werden auf die Mischung gesprüht. Das entstandene Granulat wird getrocknet und gesiebt. 80 g Talkum, 25 g Magnesiumstearat und 41 g Siliziumdioxid werden zum Rohgranulat gegeben und bis zur Homogenität gemischt. Auf einer Rundlauftablettenpresse werden bei einer Preßkraft von 10 - 30 kN Komprimate mit einer Sollmasse von 900 mg hergestellt.

### Beispiel 5

In einem Mischer werden PETN und galenische Hilfsstoffe in definierten Mengen homogen gemischt. Das Mischgut wird auf einer Tablettenpresse zu Komprimaten verarbeitet (Tabelle 1).

### Beispiel 6

Die Stoffe PETN und in definierten Mengen galenische Hilfsstoffe werden in einem Mischer bis zur Homogenität gemischt und anschließend (A) in Sachets und (B) in Kapseln gefüllt (Tabelle 2).

### Beispiel 7

In einem Mischer werden PETN und eine definierte Menge galenischer Hilfsstoffe gemischt. Anschließend erfolgt eine Kompaktierung. Die Komprimate werden mit einer Siebmaschine auf eine einheitliche Teilchengröße homogenisiert. Das Siebgut wird (A) in Sachets und (B) in Kapseln gefüllt (Tabelle 3).

### Beispiel 8

In einem Wirbelschichtgranulator werden PETN und definierte Mengen galenischer Hilfsstoffe gemischt und anschließend mit einer wäßrigen Bindemittellösung granuliert. Das getrocknete Granulat wird gesiebt und mit galenischen Fließregulier-, Schmier- und Gleitmitteln gemischt. Auf einer Tablettenpresse werden Komprimate hergestellt (A). Die so erhaltenen Komprimate werden (B) in einer Coatinganlage befilmt (Tabelle 4).

### Beispiel 9

Klinische Untersuchungen mit einmal täglichen Dosierungen von PETN größer 80 mg betegen, dass die sonst bei Nitraten auftretenden Nebenwirkungen, insbesondere
a) der typische Nitratkopfschmerz, nicht zu beobachten sind (Jähnchen et al., Pentaerythrityltetranitrat-Strukturchemische, zellbiologische und klinische Perspektiven, Dr. Dietrich Steinkopff Verlag, Darmstadt, 1997);
b) außerdem die unter PETN und in Dosen größer 80 mg ausbleibende Wirkabschwächung (Nitrattoleranz) nicht auftritt (Schneider, Pentaerythrityltetranitrat-Beiträge zum klinischen und pharmakologischen Status, Dr. Dietrich Steinkopff Verlag, Darmstadt, 1995). Das klinische Untersuchungsprogramm umfaßt des weiteren die in den oben beschriebenen Anwendungsgebieten relevanten Meßparameter, wie
c) Reduktion der Angina pectoris Anfälle (Schneider, Pentaerythrityltetranitrat-Beiträge zum klinischen und pharmakologischen Status, Dr. Dietrich Steinkopff Verlag, Darmstadt, 1995).
d) Verminderung des Akut-Nitrat-Verbrauches (Mutschler, Pentaerythrityltetranitrat-Experimentelle und klinische Befunde zu Koronarer Herzkrankheit und Herzinsuffizienz, Dr. Dietrich Steinkopff Verlag, Darmstadt, 1996) und der
e) Verlängerung und Steigerung der Belastungstoleranz (Mutschler, Pentaerythrityltetranitrat-Experimentelle und klinische Befunde zu Koronarer Herzkrankheit und Herzinsuffizienz, Dr. Dietrich Steinkopff Verlag, Darmstadt, 1996).

Hinsichtlich dieser Therapieerfolgskriterien zeigt sich bei der einmal täglichen Applikation kein Nachteil gegenüber konventionellen mehrfach täglichen Dosisregimen.

### Beispiel 10

In einer multizentrischen, randomisierten Studie wird die Behandlung der Angina pectoris mittels 150 mg PETN (analog Beispiel 8, Tabelle 4, c)) einmal täglich und mittels 50 mg PETN dreimal täglich über eine Behandlungsdauer von 2 Wochen verglichen. Die Wirksamkeit und Verträglichkeit von 150 mg/die PETN bei einmal täglichem Einnahmemodus versus dreimal täglichem Einnahmemodus und Placebo wird bewiesen.

**Tabelle 1:**

| Stoffe | a) [mg] | b) [mg] | c) [mg] | d) [mg] |
|---|---|---|---|---|
| PETN | 85 | 160 | 300 | 200 |
| Laktose | 100 | 300 | 200 | 0 |
| Cellulose | 50 | 80 | 225 | 300 |
| Stärke. | 100 | 76 | 150 | 100 |
| Talkum | 0 | 20 | 5 | 0 |
| Siliziumdioxid | 40 | 20 | 15 | 20 |
| Magnesiumstearat | 0 | 4 | 0 | 10 |
| Stearinsäure | 5 | 0 | 5 | 0 |
| Sollmasse | 380 | 660 | 900 | 630 |

**Tabelle 2:**

| Stoffe | (A) | | (B) | |
|---|---|---|---|---|
| | a) [mg] | b) [mg] | c) [mg] | d) [mg] |
| PETN | 600 | 500 | 100 | 200 |
| Laktose | 300 | 0 | 60 | 200 |
| Cellulose | 200 | 250 | 0 | 0 |
| Stärke | 250 | 250 | 60 | 0 |
| Siliziumdioxid | 50 | 5 | 5 | 5 |
| Sollmasse | 1400 | 1005 | 225 | 405 |
| Kapselgröße | - | - | 1 | 0 |

**Tabelle 3:**

| Stoffe | (A) | (B) |
|---|---|---|
| | a) [mg] | b) [mg] |
| PETN | 150 | 180 |
| Laktose | 300 | 0 |
| Cellulose | 300 | 500 |
| Stärke | 300 | 400 |
| Sollmasse | 1050 | 1080 |

**Tabelle 4:**

| Stoffe | (A) | | | | (B) |
|---|---|---|---|---|---|
| | a) [mg] | b) [mg] | c) [mg] | d) [mg] | e) [mg] |
| PETN | 100 | 125 | 150 | 100 | 120 |
| Cellulose | 0 | 210 | 200 | 205 | 220 |
| Laktose | 515 | 210 | 300 | 150 | 200 |
| Stärke | 15 | 226 | 250 | 200 | 200 |
| Gelatine | 0 | 10 | 0 | 0 | 15 |
| Hydroxypropylcellulose | 0 | 0 | 0 | 15 | 0 |
| Sorbit | 0 | 5 | 0 | 0 | 10 |
| Poly(1-vinyl-2-pyrrolidon) | 0 | 0 | 25 | 0 | 0 |
| Talkum | 15 | 21 | 5 | 10 | 15 |
| Magnesiumstearat | 5 | 7 | 0 | 0 | 5 |
| Siliziumdioxid | 10 | 11 | 15 | 15 | 15 |
| Stearinsäure | 0 | 0 | 5 | 5 | 0 |
| Sollmasse | 660 | 825 | 950 | 700 | 800 |
| Methylhydroxypropylcellulose | 0 | 0 | 0 | 0 | 4 |
| Macrogol 4000 | 0 | 0 | 0 | 0 | 4 |

## Patentansprüche

1. Pharmazeutische Zubereitungen, **dadurch gekennzeichnet, dass** sie
a) als Arzneistoff Pentaerythrityltetra-, Pentaerythrityltri-, Pentaerythrityldi- oder Pentaerythritylmononitrat,
b) in einer Menge von mehr als 80 bis 600 mg sowie
c) übliche galenische Hilfsstoffe enthalten.

2. Pharmazeutische Zubereitungen nach Anspruch 1, **dadurch gekennzeichnet, dass** die üblichen galenischen Hilfsstoffe keine retardierende Wirkung besitzen oder keine retardierende Wirkung entfalten.

3. Pharmazeutische Zubereitungen nach Anspruch 2, **dadurch gekennzeichnet, dass** die üblichen galenischen Hilfsstoffe keine retardierende Wirkung besitzen.

4. Pharmazeutische Zubereitungen nach Anspruch 2, **dadurch gekennzeichnet, dass** die üblichen galenischen Hilfsstoffe in Mengen eingesetzt werden, die nicht retardierend wirken.

5. Pharmazeutische Zubereitungen nach Anspruch 1 bis 4, **dadurch gekennzeichnet, dass** sie den Arzneistoff in einer Menge von mehr als 80 bis 500 mg enthalten.

6. Pharmazeutische Zubereitungen nach Anspruch 5, **dadurch gekennzeichnet, dass** sie den Arzneistoff in einer Menge von 85 bis 500 mg enthalten.

7. Pharmazeutische Zubereitungen nach Anspruch 6, **dadurch gekennzeichnet, dass** sie den Arzneistoff in einer Menge von 100 bis 400 mg enthalten.

8. Pharmazeutische Zubereitungen nach Anspruch 7, **dadurch gekennzeichnet, dass** sie den Arzneistoff in einer Menge von 100 bis 200 mg enthalten.

9. Pharmazeutische Zubereitungen nach Anspruch 8, **dadurch gekennzeichnet, dass** sie den Arzneistoff in einer Menge von 120 bis 180 mg enthalten.

10. Pharmazeutische Zubereitungen nach Anspruch 9, **dadurch gekennzeichnet, dass** sie den Arzneistoff in einer Menge von 150 mg enthalten.

11. Pharmazeutische Zubereitungen nach Anspruch 1 bis 10, **dadurch gekennzeichnet, dass** sie den Arzneistoff in einer Menge von bis zu 50 Masse-% enthalten.

12. Pharmazeutische Zubereitungen nach Anspruch 11, **dadurch gekennzeichnet, dass** sie den Arzneistoff in einer Menge von 10 bis 50 Masse-% enthalten.

13. Pharmazeutische Zubereitungen nach Anspruch 1 bis 12, **dadurch gekennzeichnet, dass** sie den Arzneistoff homogen verteilt enthalten.

14. Pharmazeutische Zubereitungen nach Anspruch 1 bis 13, **dadurch gekennzeichnet, dass** sie als feste Peroralia vorliegen.

15. Pharmazeutische Zubereitungen nach Anspruch 14, **dadurch gekennzeichnet, dass** sie in Form von Pulvern, Granulaten, Pellets, Tabletten, Filmtabletten, Kapseln und Dragees vorliegen.

16. Pharmazeutische Zubereitungen nach Anspruch 15, **dadurch gekennzeichnet, dass** sie in Form von Tabletten, Filmtabletten und Dragees vorliegen

17. Pharmazeutische Zubereitungen nach Anspruch 16, **dadurch gekennzeichnet, dass** sie mit einem, insbesondere magensaftresistenten, Überzug oder Film versehen sind.

18. Verfahren zur Herstellung von pharmazeutischen Zubereitungen, **dadurch gekennzeichnet, dass** die Arzneistoffe Pentaerythrityltetra-, Pentaerythrityltri-, Pentaerythrityldi- oder Pentaerythritylmononitrat,
a) in einer definierten Menge mit üblichen galenischen Hilfsstoffen gemischt,
b) die erhaltenen Mischungen
i) in Kapseln oder Beuteln abgefüllt,
ii) kompaktiert, anschließend vermahlen und homogenisiert, in Kapseln oder Beuteln abgefüllt sowie zu Tabletten oder Drageekernen verpreßt,
iii) granuliert, anschließend in Kapseln oder Beuteln abgefüllt sowie zu Tabletten oder Drageekernen verpreßt werden, so dass
c) die pharmazeutischen Zubereitungen mehr als 80 bis 600 mg Arzneistoff enthalten, welche
d) bei Bedarf befilmt oder überzogen werden.

19. Verfahren nach Anspruch 18, **dadurch gekennzeichnet, dass** das Verpressen zu Tabletten oder Drageekernen durch Direkttablettierung erfolgt.

20. Verfahren nach Anspruch 18 und 19, **dadurch gekennzeichnet, dass** das Verpressen zu Tabletten oder Drageekernen bei Preßdrucken bis 50 kN, insbesondere bei 10 bis 30 kN, erfolgt.

21. Verfahren nach Anspruch 18, **dadurch gekennzeichnet, dass** das Granulieren mit einer wäßrigen Bindemittellösung erfolgt.

22. Pharmazeutische Zubereitungen nach Anspruch 1 bis 17 zur Anwendung in einem Verfahren zur therapeutischen und protektiven Behandlung des menschlischen Organ- und Gefäßsytems.

23. Pharmazeutische Zubereitungen nach Anspruch 22 zur Anwendung in einem Verfahren zur Protektion von Gefäßen und Geweben.

24. Pharmazeutische Zubereitungen nach Anspruch 22 zur Anwendung in einem Verfahren zur Therapie von Herz-, Kreislauf- oder cerebrovaskulären Erkrankungen.

25. Pharmazeutische Zubereitungen nach Anspruch 24 zur Anwendung in einem Verfahren zur therapeutischen Behandlung von dass Angina pectoris, ischämischer Herzkrankheit, Postmyokardinfarkt oder chronischer Herzinsuffizienz.

26. Pharmazeutische Zubereitungen nach den Ansprüchen 22 bis 25 zur einmal täglichen Applikation in einem Verfahren zur therapeutischen und protektiven Behandlung des menschlischen Organ- und Gefäßsytems.

27. Pharmazeutische Zubereitungen nach den Ansprüchen 22 bis 26 zur Langzeittherapie zur Anwendung in einem Verfahren zur therapeutischen und protektiven Behandlung des menschlischen Organ- und Gefäßsytems.

28. Pharmazeutische Zubereitungen nach den Ansprüchen 22 bis 27 zur Anwendung in einem Verfahren zur therapeutischen und protektiven Behandlung des menschlischen Organ- und Gefäßsytems, **dadurch gekennzeichnet, dass** andere zur Behandlung von Herz-/Kreislauferkrankungen angewandten Arzneistoffe, insbesondere solche aus den Indikationsgruppen der ACE-Hemmer, Antiatherosklerotika, Antihypertensiva, Betablocker, Cholesterinsenker, Diuretika, Kalziumantagonisten, Koronardilatatoren, Lipidsenker, peripheren Vasodilatatoren oder Thrombozytenaggregationshemmer enthalten sind.

29. Pharmazeutische Pentaerythrityltetra-, Pentaerythrityltri-, Pentaerythrityldi- oder Pentaerythritylmononitrat enthaltende Zubereitungen zur Anwendung in einem Verfahren zur therapeutischen und protektiven Behandlung des menschlischen Organ- und Gefäßsytems, **dadurch gekennzeichnet, dass** mehr als 80 mg bis 600 mg Arzneistoff unter Verzicht auf galenische Retardierungsprizipien in der Zubereitung enthalten sind.

30. Pharmazeutische Zubereitungen nach Anspruch 29 zur Anwendung in einem Verfahren zur zur Protektion von Gefäßen und Geweben.

31. Pharmazeutische Zubereitungen nach Anspruch 29 zur Anwendung in einem Verfahren zur Therapie von Herz-, Kreislauf- oder cerebrovaskulären Erkrankungen.

32. Pharmazeutische Zubereitungen nach nach Anspruch 31 zur Anwendung in einem Verfahren zur therapeutischen Behandlung von dass Angina pectoris, ischämischer Herzkrankheit, Postmyokardinfarkt oder chronischer Herzinsuffizienz.

33. Pharmazeutische Zubereitungen nach den Ansprüchen 29 bis 32 zur Anwendung in einem Verfahren zur therapeutischen und protektiven Behandlung des menschlischen Organ- und Gefaßsytems, **dadurch gekennzeichnet, dass** andere zur Behandlung von Herz-/Kreislauferkrankungen angewandten Arzneistoffe, insbesondere solche aus den Indikationsgruppen der ACE-Hemmer, Antiatherosklerotika, Antihypertensiva, Betablocker, Cholesterinsenker, Diuretika, Kalziumantagonisten, Koronardilatatoren, Lipidsenker, peripheren Vasodilatatoren oder Thrombozytenaggregationshemmer enthalten sind.

34. Pharmazeutische Zubereitungen nach den Ansprüchen 29 bis 33 zur einmal täglichen Applikation in einem Verfahren zur therapeutischen und protektiven Behandlung des menschlischen Organ- und Gefäßsytems.

35. Pharmazeutische Zubereitungen nach den Ansprüchen 29 bis 34 zur Anwendung in einem Langzeittherapie- Verfahren zur Behandlung des menschlischen Organ- und Gefäßsytems.

## Claims

1. Pharmaceutical formulations, **characterized in that** they comprise
a) pentaerythrityl tetra-, pentaerythrityl tri-, pentaerythrityl di- or pentaerythrityl mononitrate as the medicinal substance
b) in an amount of more than 80 to 600 mg and
c) conventional galenical auxiliaries.

2. Pharmaceutical formulations according to claim 1, **characterized in that** the conventional galenical auxiliaries have no retarding action or unfold no retarding action.

3. Pharmaceutical formulations according to claim 2, **characterized in that** the conventional galenical auxiliaries have no retarding action.

4. Pharmaceutical formulations according to claim 2, **characterized in that** the conventional galenical auxiliaries are employed in amounts which do not have a retarding action.

5. Pharmaceutical formulations according to claim 1 to 4, **characterized in that** they comprise the medicinal substance in an amount of more than 80 to 500 mg.

6. Pharmaceutical formulations according to claim 5, **characterized in that** they comprise the medicinal substance in an amount of 85 to 500 mg.

7. Pharmaceutical formulations according to claim 6, **characterized in that** they comprise the medicinal substance in an amount of 100 to 400 mg.

8. Pharmaceutical formulations according to claim 7, **characterized in that** they comprise the medicinal substance in an amount of 100 to 200 mg.

9. Pharmaceutical formulations according to claim 8, **characterized in that** they comprise the medicinal substance in an amount of 120 to 180 mg.

10. Pharmaceutical formulations according to claim 9, **characterized in that** they comprise the medicinal substance in an amount of 150 mg.

11. Pharmaceutical formulations according to claim 1 to 10, **characterized in that** they comprise the medicinal substance in an amount of up to 50 % by weight.

12. Pharmaceutical formulations according to claim 11, **characterized in that** they comprise the medicinal substance in an amount of 10 to 50 % by weight.

13. Pharmaceutical formulations according to claim 1 to 12, **characterized in that** they comprise the medicinal substance in homogeneous distribution.

14. Pharmaceutical formulations according to claim 1 to 13, **characterized in that** they are in the form of solid compositions for peroral administration.

15. Pharmaceutical formulations according to claim 14, **characterized in that** they are in the form of powders, granules, pellets, tablets, film-covered tablets, capsules and coated tablets.

16. Pharmaceutical formulations according to claim 15, **characterized in that** they are in the form of tablets, film-covered tablets and coated tablets.

17. Pharmaceutical formulations according to claim 16, **characterized in that** they are provided with a coating or film, in particular one resistant to gastric juices.

18. Process for the preparation of pharmaceutical formulations, **characterized in that** the medicinal substances pentaerythrityl tetra-, pentaerythrityl tri-, pentaerythrityl di- or pentaerythrityl mononitrate
a) are mixed in a defined amount with conventional galenical auxiliaries,
b) the mixtures obtained are
i) filled into capsules or bags,
ii) compacted, subsequently ground and homogenized, filled into capsules or bags and pressed to tablets or coated tablet cores,
iii) granulated, subsequently filled into capsules or bags and pressed to tablets or coated tablet cores, so that
c) the pharmaceutical formulations comprise more than 80 to 600 mg of medicinal substance, and these
d) are covered with film or coated as required.

19. Process according to claim 18, **characterized in that** the pressing to tablets or coated tablet cores is carried out by direct tablet-making.

20. Process according to claim 18 and 19, **characterized in that** the pressing to tablets or coated tablet cores is carried out under pressing pressures of up to 50 kN, in particular at 10 to 30 kN.

21. Process according to claim 18, **characterized in that** the granulation is carried out with an aqueous binder solution.

22. Pharmaceutical formulations according to claim 1 to 17 for use in a method for therapeutic and protective treatment of the human organ and vascular system.

23. Pharmaceutical formulations according to claim 22 for use in a method for protection of vessels and tissues.

24. Pharmaceutical formulations according to claim 22 for use in a method for therapy of cardiac, circulatory or cerebrovascular diseases.

25. Pharmaceutical formulations according to claim 24 for use in a method for therapeutic treatment of angina pectoris, ischaemic heart disease, postmyocardial infarction or chronic cardiac insufficiency.

26. Pharmaceutical formulations according to claims 22 to 25 for once-daily administration in a method for therapeutic and protective treatment of the human organ and vascular system.

27. Pharmaceutical formulations according to claims 22 to 26 for long-term therapy for use in a method for therapeutic and protective treatment of the human organ and vascular system.

28. Pharmaceutical formulations according to claims 22 to 27 for use in a method for therapeutic and protective treatment of the human organ and vascular system, **characterized in that** they comprise other medicinal substances used for treatment of cardiac/circulatory diseases, in particular those from the indication groups of ACE inhibitors, antiatherosclerotics, antihypertensives, beta blockers, cholesterol-lowering agents, diuretics, calcium antagonists, coronary dilators, lipid-lowering agents, peripheral vasodilators or platelet aggregation inhibitors.

29. Pharmaceutical formulations comprising pentaerythrityl tetra-, pentaerythrityl tri-, pentaerythrityl di- or pentaerythrityl mononitrate for use in a method for therapeutic and protective treatment of the human organ and vascular system, **characterized in that** the formulation comprises more than 80 mg to 600 mg of medicinal substance, without the use of galenical retarding principles.

30. Pharmaceutical formulations according to claim 29 for use in a method for protection of vessels and tissues.

31. Pharmaceutical formulations according to claim 29 for use in a method for therapy of cardiac, circulatory or cerebrovascular diseases.

32. Pharmaceutical formulations according to claim 31 for use in method for therapeutic treatment of angina pectoris, ischaemic cardiac disease, postmyocardial infarction or chronic cardiac insufficiency.

33. Pharmaceutical formulations according to claims 29 to 32 for use in a method for therapeutic and protective treatment of the human organ and vascular system, **characterized in that** they comprise other medicinal substances used for treatment of cardiac/circulatory diseases, in particular those from the indication groups of ACE inhibitors, antiatherosclerotics, antihypertensives, beta blockers, cholesterol-lowering agents, diuretics, calcium antagonists, coronary dilators, lipid-lowering agents, peripheral vasodilators or platelet aggregation inhibitors.

34. Pharmaceutical formulations according to claims 29 to 33 for once-daily administration in a method for therapeutic and protective treatment of the human organ and vascular system.

35. Pharmaceutical formulations according to claims 29 to 34 for use in a long-term therapy method for treatment of the human organ and vascular system.

## Revendications

1. Formulations pharmaceutiques, **caractérisées par le fait qu'**elles contiennent
a) du pentaérythrityl tétra-, pentaérythrityl tri-, pentaérythrityl di- ou pentaérythrityl mononitrate en tant que substance médicinale
b) en une quantité de plus de 80 à 600 mg et
c) des auxiliaires galéniques traditionnels.

2. Formulations pharmaceutiques selon la revendication 1, **caractérisées par le fait que** les auxiliaires galéniques traditionnels n'ont aucun effet retard ou ne dévoilent aucun effet retard.

3. Formulations pharmaceutiques selon la revendication 2, **caractérisées par le fait que** les auxiliaires galéniques traditionnels n'ont aucun effet retard.

4. Formulations pharmaceutiques selon la revendication 2, **caractérisées par le fait que** les auxiliaires galéniques traditionnels sont employés en des quantités qui n'ont aucun effet retard.

5. Formulations pharmaceutiques selon la revendication 1 à 4, **caractérisées par le fait qu'**elles contiennent une quantité de plus de 80 à 500 mg de substance médicinale.

6. Formulations pharmaceutiques selon la revendication 5, **caractérisées par le fait qu'**elles contiennent une quantité de 85 à 500 mg de substance médicinale.

7. Formulations pharmaceutiques selon la revendication 6, **caractérisées par le fait qu'**elles contiennent une quantité de 100 à 400 mg de substance médicinale.

8. Formulations pharmaceutiques selon la revendication 7, **caractérisées par le fait qu'**elles contiennent une quantité de 100 à 200 mg de substance médicinale.

9. Formulations pharmaceutiques selon la revendication 8, **caractérisées par le fait qu'**elles contiennent une quantité de 120 à 180 mg de substance médicinale.

10. Formulations pharmaceutiques selon la revendication 9, **caractérisées par le fait qu'**elles contiennent une quantité de 150 mg de substance médicinale.

11. Formulations pharmaceutiques selon la revendication 1 à 10, **caractérisées par le fait qu'**elles contiennent une quantité maximale de 50 % en poids de substance médicinale.

12. Formulations pharmaceutiques selon la revendication 11, **caractérisées par le fait qu'**elles contiennent une quantité de 10 à 50 % en poids de substance médicinale.

13. Formulations pharmaceutiques selon la revendication 1 à 12, **caractérisées par le fait qu'**elles contiennent la substance médicinale en une distribution homogène.

14. Formulations pharmaceutiques selon la revendication 1 à 13, **caractérisées par le fait qu'**elles se présentent sous la forme de compositions solides à administrer par voie orale.

15. Formulations pharmaceutiques selon la revendication 14, **caractérisées par le fait qu'**elles se présentent sous la forme de poudres, de granulés, de pellets, de comprimés, de comprimés pelliculés, de gélules et de comprimés enrobés.

16. Formulations pharmaceutiques selon la revendication 15, **caractérisées par le fait qu'**elles se présentent sous la forme de comprimés, de comprimés pelliculés et de comprimés enrobés.

17. Formulations pharmaceutiques selon la revendication 16, **caractérisées par le fait qu'**elles sont fournies avec un enrobage ou une pellicule, en particulier une pellicule ou un enrobage gastrorésistant.

18. Procédé de préparation de formulations pharmaceutiques, **caractérisé par le fait que** les substances médicinales pentaérythrityl tétra-, pentaérythrityl tri-, pentaérythrityl di- ou pentaérythrityl mononitrate
a) sont mélangées en une quantité définie aux auxiliaires galéniques traditionnels,
b) les mélanges obtenus sont
i) versés dans des gélules ou des sachets,
ii) tassés, puis broyés et homogénéisés,
versés dans des gélules ou des sachets et pressés pour former des comprimés ou des noyaux de comprimés enrobés,
iii) granulés, puis
versés dans des gélules ou des sachets et pressés pour former des comprimés ou des noyaux de comprimés enrobés, afin que
c) les formulations pharmaceutiques contiennent plus de 80 à 600 mg de substance médicinale, et que ces comprimés
d) soient recouverts d'une pellicule ou d'un enrobage, suivant le cas.

19. Procédé selon la revendication 18, **caractérisé par le fait que** la compression pour former des comprimés ou des noyaux de comprimés enrobés s'effectue selon une méthode directe de fabrication des comprimés.

20. Procédé selon la revendication 18 et la revendication 19, **caractérisé par le fait que** la compression pour former des comprimés ou des noyaux de comprimés enrobés s'effectue sous des pressions de compression pouvant aller jusqu'à 50 kN, en particulier de 10 à 30 kN.

21. Procédé selon la revendication 18, **caractérisé par le fait que** la granulation s'effectue avec un liant en solution aqueuse.

22. Formulations pharmaceutiques selon la revendication 1 à 17 à utiliser dans une méthode pour le traitement thérapeutique et protecteur du système organique et vasculaire de l'homme.

23. Formulations pharmaceutiques selon la revendication 22 à utiliser dans une méthode pour la protection des vaisseaux et des tissus.

24. Formulations pharmaceutiques selon la revendication 22 à utiliser dans une méthode pour la thérapie des maladies cardiaques, circulatoires ou cérébrovasculaires.

25. Formulations pharmaceutiques selon la revendication 24 à utiliser dans une méthode pour le traitement thérapeutique de l'angine de poitrine, de la cardiopathie ischémique, à la suite d'un infarctus du myocarde ou de l'insuffisance cardiaque chronique.

26. Formulations pharmaceutiques selon les revendications 22 à 25 à administrer une fois par jour dans une méthode pour le traitement thérapeutique et protecteur du système organique et vasculaire de l'homme.

27. Formulations pharmaceutiques selon les revendications 22 à 26 pour la thérapie à long terme à utiliser dans une méthode pour le traitement thérapeutique et protecteur du système organique et vasculaire de l'homme.

28. Formulations pharmaceutiques selon les revendications 22 à 27 à utiliser dans une méthode pour le traitement thérapeutique et protecteur du système organique et vasculaire de l'homme, **caractérisées par le fait qu'**elles contiennent d'autres substances médicinales utilisées pour le traitement des maladies cardiaques/circulatoires, en particulier celles pour lesquelles les groupes de médicaments suivants sont indiqués : inhibiteurs ECA, antiathéroscléreux, antihypertenseurs, bêta-bloquants, hypocholestérolémiants, diurétiques, inhibiteurs calciques, dilatateurs coronaires, hypolipidémiants, vasodilatateurs périphériques ou inhibiteurs de l'agrégation plaquettaire.

29. Formulations pharmaceutiques contenant du pentaérythrityl tétra-, pentaérythrityl tri-, pentaérythrityl di- ou pentaérythrityl mononitrate à utiliser dans une méthode pour le traitement thérapeutique et protecteur du système organique et vasculaire de l'homme, **caractérisées par le fait que** la formulation contient plus de 80 mg à 600 mg de substance médicinale, mais est exempte de principes galéniques à effet retard quelconques.

30. Formulations pharmaceutiques selon la revendication 29 à utiliser dans une méthode pour la protection des vaisseaux et des tissus.

31. Formulations pharmaceutiques selon la revendication 29 à utiliser dans une méthode pour la thérapie des maladies cardiaques, circulatoires ou cérébrovasculaires.

32. Formulations pharmaceutiques selon la revendication 31 à utiliser dans une méthode pour le traitement thérapeutique de l'angine de poitrine, de la cardiopathie ischémique, à la suite d'un infarctus du myocarde ou de l'insuffisance cardiaque chronique.

33. Formulations pharmaceutiques selon les revendications 29 à 32 à utiliser dans une méthode pour le traitement thérapeutique et protecteur du système organique et vasculaire de l'homme, **caractérisées par le fait qu'**elles contiennent d'autres substances médicinales utilisées pour le traitement des maladies cardiaques/circulatoires, en particulier celles pour lesquelles les groupes de médicaments suivants sont indiqués : inhibiteurs ECA, antiathéroscléreux, antihypertenseurs, bêta-bloquants, hypocholestérolémiants, diurétiques, inhibiteurs calciques, dilatateurs coronaires, hypolipidémiants, vasodilatateurs périphériques ou inhibiteurs de l'agrégation plaquettaire.

34. Formulations pharmaceutiques selon les revendications 29 à 33 à administrer une fois par jour dans une méthode pour le traitement thérapeutique et protecteur du système organique et vasculaire de l'homme.

35. Formulations pharmaceutiques selon les revendications 29 à 34 à utiliser dans une méthode de thérapie à long terme pour le traitement du système organique et vasculaire de l'homme.
